# FASCICULE DE BREVET EUROPEEN

(11) **EP 2 594 209 B1**
(45) Date de publication et mention de la délivrance du brevet: **30.04.2014**
(21) Numéro de dépôt: 12192593.7
(22) Date de dépôt: 14.11.2012
(51) Int. Cl.: A61B 17/15

(54) **Instrumentation chirurgicale spécifique à un patient pour la préparation d'un os du patient**
Patientenspezifische chirurgische Instrumente für die Vorbereitung eines Knochens des Patienten
Patient-specific surgical instrument for preparing a bone of the patient

(30) Priorité: 15.11.2011 FR 1160368
(43) Date de publication de la demande: 22.05.2013
(73) Titulaire: Tornier, 38330 Montbonnot-Saint-Martin (FR)
(72) Inventeur: Zakaria, Toufik, 38000 Grenoble (FR); Lizee, Emmanuel, 38330 Saint Ismier (FR)
(74) Mandataire: Grand, Guillaume

(56) Documents cités:
- US-A1- 2008 161 815
- US-A1- 2009 087 276
- US-A1- 2010 087 829
- US-A1- 2011 071 533

## Description

La présente invention concerne une instrumentation chirurgicale spécifique à un patient, pour la préparation d'un os de ce patient, par exemple un fémur, en vue d'y implanter un composant de prothèse.

Le domaine de l'invention est celui des instrumentations dites « sur-mesure » ou « personnalisées », en lien avec un patient précis, sur lequel l'instrumentation est destinée à être exclusivement utilisée par un chirurgien. Ce genre d'instrumentation spécifique à un patient s'oppose aux instrumentations standards, qui sont utilisées indifféremment sur différents patients, le cas échéant en étant réutilisées plusieurs fois de manière successive, en étant nettoyées et stérilisées entre chaque utilisation.

L'avènement des instrumentations « sur-mesure » est lié aux possibilités actuelles d'acquérir des données pré-opératoires suffisamment précises afin de concevoir, notamment du point de vue dimensionnel, des instruments dont les interfaces de coopération mécanique avec les os du patient sont spécifiquement définies en tenant compte de la forme précise, notamment des reliefs de surface, de ces os. Les données pré-opératoires utilisées proviennent typiquement d'images scanner ou, plus généralement, de tout enregistrement de données de cartographie osseuse avantageusement obtenues de manière non invasive. Ces données sont traitées par ordinateur afin de commander la fabrication d'instruments chirurgicaux sur mesure, une fois que le chirurgien a décidé les détails de la procédure chirurgicale qu'il va suivre pas-à-pas lors d'une intervention à venir.

Lors de l'intervention, le chirurgien positionne sur l'os un bloc de guidage, comportant une surface conformée de manière spécifiquement ajustée à l'os du patient. Le bloc de guidage permet alors de guider l'application sur l'os d'un outil de préparation osseuse, tel qu'un foret de perçage ou une broche d'ancrage. Par la suite, le bloc de guidage doit être retiré ou écarté de l'os pour permettre le positionnement d'un bloc de coupe. Comme la forme du bloc de guidage épousant l'os est relativement complexe, cela le rend difficile, voire impossible, à retirer de l'os lorsque les broches y sont insérées.

US-A-2007/288030 décrit une instrumentation fémorale spécifique à un patient, comportant un bloc fémoral délimitant une surface d'appui fixe spécifiquement ajustée sur le fémur. Le bloc comporte plusieurs orifices traversants, prévus pour recevoir des broches utilisées pour le positionnement de guides de coupe. Deux orifices ménagés dans le bloc sont ouverts dans une direction transverse à la direction d'insertion des broches. Ainsi, les orifices ouverts sur le bloc permettent au chirurgien de retirer ce bloc sans avoir à retirer des broches qui y sont insérées. La forme particulière de ces orifices ouverts implique néanmoins que le guidage d'un outil de perçage de trous, et/ou l'insertion d'une broche, dans l'os, peuvent manquer de précision. En outre, le positionnement des broches est nécessairement limité à certaines configurations pour permettre le retrait du bloc.

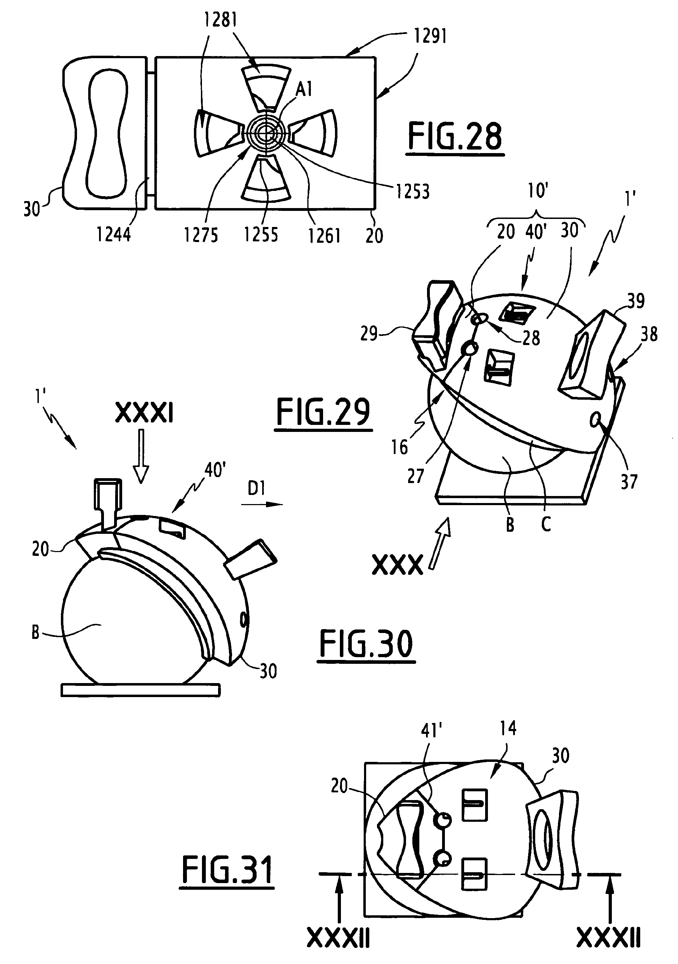

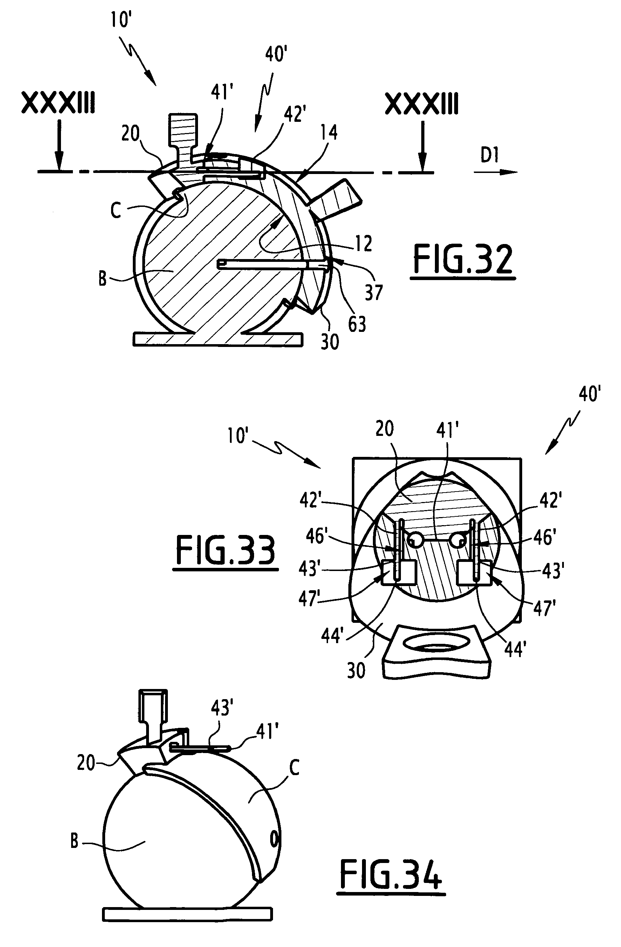

US-A-2009/087276 décrit une instrumentation chirurgicale spécifique à un patient, correspondant au préambule de la revendication 1. Dans le mode de réalisation des figures 31 et 32, l'instrumentation comprend des broches de fixation insérées dans des ouvertures traversant une partie principale et une partie secondaire du bloc. Si un tel assemblage est trop rigide, il est difficile à manipuler par le chirurgien. A l'inverse un tel assemblage peut comporter du jeu, ce qui réduit la précision de positionnent des parties du bloc. Dans ce cas les coupes sont moins précises et la prothèse peut être mal positionnée, ce qui risque d'entraîner une révision précoce. Comme la partie principale, la partie secondaire et les broches sont quatre pièces distinctes, il existe un risque de perte de pièces lors de la manipulation et l'instrumentation est peu pratique à mettre en oeuvre.

Le but de l'invention est de proposer une instrumentation chirurgicale spécifique améliorée, procurant un gain de temps et de précision au chirurgien.

A cet effet, l'invention a pour objet une instrumentation chirurgicale spécifique à un patient pour la préparation d'un os du patient, l'instrumentation comprenant un bloc spécifique au patient délimitant une surface d'appui fixe sur l'os, qui est conformée de manière spécifiquement ajustée à cet os, et incluant une partie principale qui délimite en partie la surface d'appui fixe et qui délimite au moins deux orifices primaires de passage respectif d'une broche primaire, ces orifices primaires traversant le bloc entre la surface d'appui et une surface opposée du bloc, l'instrumentation comprenant également :
- une partie secondaire qui appartient au bloc, qui délimite en partie la surface d'appui et qui comporte au moins deux orifices secondaires de passage respectif d'un outil de préparation osseuse, tel qu'un foret de perçage ou une broche d'ancrage, ces orifices secondaires traversant le bloc entre la surface d'appui et la surface opposée du bloc, et
- des moyens de dégagement mécanique de la partie secondaire par rapport à la partie principale, adaptés pour, alors que la partie principale est fixée sur l'os par des broches primaires passées dans les orifices primaires, rendre la partie secondaire amovible par rapport à la partie principale.

L'instrumentation est **caractérisée en ce que** les moyens de dégagement mécanique comprennent au moins une cavité spécifique au patient, qui s'étend à travers le bloc, entre la partie principale et la partie secondaire, suivant une enveloppe géométrique de la surface d'appui fixe.

Ainsi, l'invention permet de faciliter les manipulations per-opératoires du chirurgien. L'instrumentation est simple et pratique d'utilisation : le bloc spécifique est adapté, d'une part, pour positionner sur l'os des broches secondaires qui seront ultérieurement utilisées pour mettre en place sur l'os un bloc de coupe ou tout autre système de préparation de l'os puis, d'autre part, pour être dégagé entièrement ou partiellement de l'os sans altérer la précision du positionnement de ces broches. Le chirurgien a simplement à casser, couper, plier, déclipser ou retirer les moyens de dégagement mécanique de la partie secondaire par rapport à la partie principale, en fonction du mode de réalisation de ces moyens. Afin d'éviter une perte de précision résultant du démontage et du remontage de broches, il est en effet particulièrement avantageux soit de guider l'application d'un foret de perçage ou similaire par la partie secondaire, avant de mettre en place les broches secondaires dans les trous ainsi percés dans l'os après dégagement de la partie secondaire, soit de laisser les broches secondaire en place dans l'os pendant le retrait, au moins partiel, du bloc de guidage. En pratique, le dégagement de la partie secondaire est réalisé selon une direction qui n'induit pas d'interférence de coincement entre la partie de la surface d'appui, délimitée par cette partie secondaire, et les reliefs du fémur auxquels est ajustée cette partie de la surface d'appui.

L'invention permet de former des zones de résistance mécanique réduite, plus facilement déformables et/ou sécables, tout en s'assurant que le bloc présente une résistance mécanique suffisante lors de son positionnement sur l'os et lors de l'insertion des broches. Avec une cavité non spécifique au patient, autrement dit une cavité générique, un tel compromis serait difficile à obtenir du fait de la géométrie complexe de l'os. D'un patient à l'autre, une instrumentation comprenant une cavité générique ne serait pas satisfaisante. En revanche, grâce à la ou les cavités spécifiques au patient l'instrumentation selon l'invention facilite les manipulations per-opératoires du chirurgien.

Selon d'autres caractéristiques avantages de l'invention, prises isolément ou en combinaison :
- Le bloc et la cavité spécifique présentent chacun une épaisseur sensiblement constante, suivant une direction normale à la surface d'appui fixe), au niveau des moyens de dégagement mécanique.
- Le bloc comporte au moins une ouïe débouchant d'une part, dans la cavité spécifique et, d'autre part, au niveau de la surface d'appui fixe ou de la surface opposée du bloc.
- Les moyens de dégagement mécanique comprennent deux cavités spécifiques au patient, qui s'étendent à travers le bloc suivant une enveloppe géométrique de la surface d'appui fixe.
- La ou chaque cavité spécifique comporte au moins une ouverture débouchant hors du bloc.
- Les moyens de dégagement mécanique sont en outre adaptés pour, alors que des broches secondaires sont passées dans les orifices secondaires, rendre la partie secondaire amovible par rapport à la partie principale par coulissement le long des broches secondaires.
- Les moyens de dégagement mécanique sont venus de matière à la fois avec la partie principale et la partie secondaire.
- Les moyens de dégagement mécanique sont venus de matière à la fois avec la partie principale et la partie secondaire lors de la fabrication du bloc par frittage laser de poudre.
- Les moyens de dégagement mécanique comprennent au moins un élément de liaison qui relie la partie principale et la partie secondaire et est destructible par rupture, par arrachement et/ou par section.
- Les moyens de dégagement mécanique comprennent au moins une ouverture de réception d'un outil au contact du ou des éléments de liaison pour la rupture, l'arrachement et/ou la section du ou des éléments de liaison.
- Le bloc et la cavité spécifique présentent chacun une épaisseur sensiblement constante, suivant une direction normale à la surface d'appui fixe, au niveau des moyens de dégagement mécanique.
- Les moyens de dégagement mécanique comprennent un élément déformable élastiquement qui relie la partie principale et la partie secondaire et autorise un dégagement réversible par pliage de la partie secondaire par rapport à la partie principale.
- Les moyens de dégagement mécanique comprennent un organe rotatif et en ce qu'une rotation de l'organe rotatif exerce des efforts sur le ou les éléments de liaison pour la rupture, l'arrachement et/ou la section du ou des éléments de liaison.
- Les moyens de dégagement mécanique sont adaptés pour successivement être solidarisés au bloc et au moins partiellement désolidarisés du bloc, notamment par clipsage/déclipsage ou par vissage/dévissage.
- Les moyens de dégagement mécanique sont au moins partiellement venus de matière avec une première partie parmi la partie principale et la partie secondaire.
- Les moyens de dégagement mécanique comportent au moins un élément d'accroche temporaire de la première partie sur une deuxième partie parmi la partie principale et la partie secondaire.

L'invention sera mieux comprise à la lecture de la description qui va suivre, donnée uniquement à titre d'exemple non limitatif et faite en référence aux dessins annexés sur lesquels :
- la figure 1 est une vue en élévation d'une instrumentation conforme à l'invention, comprenant un bloc spécifique ajusté sur un fémur gauche d'un patient, la direction d'observation de cette figure 1 correspondant à la direction longitudinale du fémur ;
- la figure 2 est une vue en perspective du bloc spécifique, légèrement par l'arrière et sans le fémur, selon la flèche II à la figure 1 ;
- la figure 3 est une vue en élévation selon la flèche III à la figure 1, le fémur n'étant pas représenté ;
- la figure 4 est une coupe selon la ligne IV-IV à la figure 2 ;
- la figure 5 est une vue à plus grande échelle du détail V à la figure 4 ;
- la figure 6 est une vue en perspective partielle d'une instrumentation;
- la figure 7 est une vue en perspective partielle d'une instrumentation;
- la figure 8 est une vue en perspective partielle d'une instrumentation;
- la figure 9 est une vue en perspective partielle d'une instrumentation;
- la figure 10 est une coupe dans le plan X à la figure 9 ;
- la figure 11 est une vue en perspective partielle d'une instrumentation, certains éléments étant montrés en transparence et représentés par des traits en pointillés ;
- la figure 12 est une coupe dans le plan médian XII à la figure 11 ;
- la figure 13 est une vue en perspective partielle d'une instrumentation;
- la figure 14 est une vue en élévation selon la flèche XIV à la figure 13 ;
- la figure 15 est une coupe selon la ligne XV-XV à la figure 12 ;
- la figure 16 est une vue en perspective partielle d'une instrumentation;
- la figure 17 est une vue en perspective partielle d'une instrumentation;
- la figure 18 est une coupe dans le plan médian XVIII à la figure 17 ;
- la figure 19 est une vue en perspective partielle d'une instrumentation ;
- la figure 20 est une coupe dans le plan médian XX à la figure 19 ;
- la figure 21 est une vue en perspective partielle d'une instrumentation ;
- la figure 22 est une vue en perspective partielle d'une instrumentation ;
- la figure 23 est une coupe dont le plan médian XXIII à la figure 22 ;
- la figure 24 est une vue en perspective partielle d'une instrumentation ;
- la figure 25 est une vue en élévation selon la flèche XXV à la figure 24, certains éléments étant montrés en transparence et représentés par des traits en pointillés ;
- la figure 26 est une coupe dans le plan XXVI à la figure 25 ;
- la figure 27 est une coupe dans le plan XXVII à la figure 26 ;
- la figure 28 est une coupe dans le plan XXVIII à la figure 25 ;
- la figure 29 est une vue en perspective d'une instrumentation,
   comprenant un bloc spécifique ajusté sur un fémur représenté schématiquement ;
- la figure 30 est une vue en élévation selon la flèche XXX à la figure 29, la direction d'observation de cette figure 30 correspondant à la direction latéro-médiale du fémur ;
- la figure 31 est une vue en élévation selon la flèche XXXI à la figure 30 ;
- la figure 32 est une vue coupe selon la ligne XXXII-XXXII à la figure 31 ;
- la figure 33 est une coupe selon la ligne XXXIII-XXXIII à la figure 32 ; et
- la figure 34 est une vue analogue à la figure 30, l'instrumentation étant partiellement représentée.

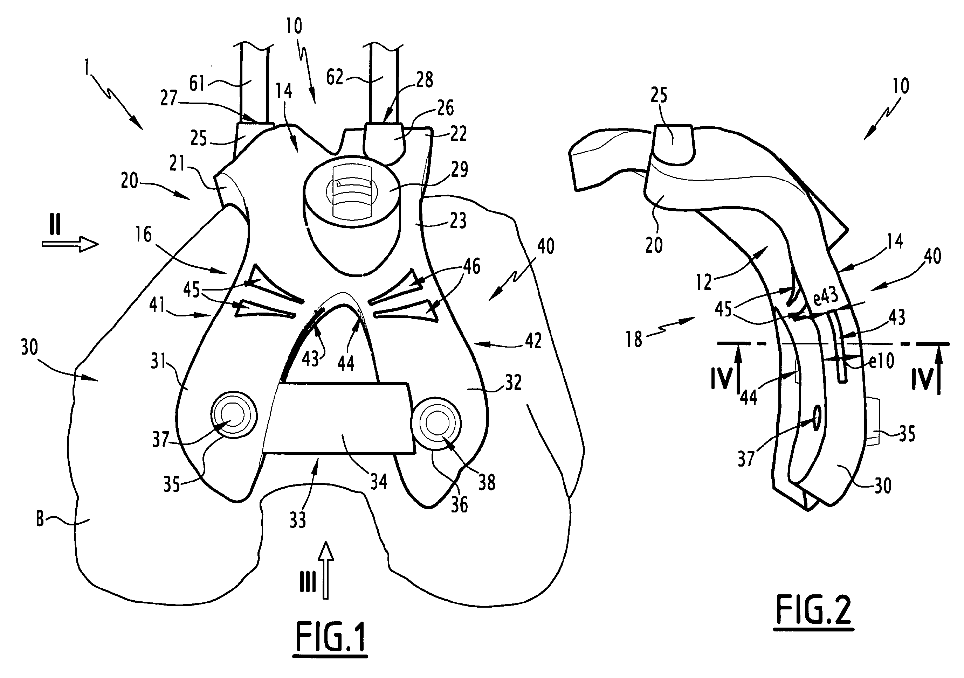

Sur les figures 1 à 5 est représentée une instrumentation chirurgicale 1 spécifique à un patient, pour la préparation d'un os B de ce patient en vue de l'implantation d'une prothèse.

Plus précisément, sur l'exemple des figures 1 à 5, l'os est le fémur droit du patient, à préparer pour l'implantation d'un composant fémoral de prothèse du genou. On comprend que l'instrumentation chirurgicale spécifique 1 selon l'invention peut être adaptée à différents ossements du corps humain ou animal.

Dans toute la suite, les termes « supérieur », « inférieur », « postérieur », etc. s'entendent dans leur sens anatomique usuel, en considérant que le patient opéré se tient debout sur une surface horizontale.

Préalablement à l'intervention chirurgicale d'implantation proprement dite, on recueille des données de cartographie relatives au fémur B du patient à opérer. En pratique, ces données de cartographie pré-opératoires peuvent être obtenues de diverses manières. A titre d'exemple, des images scanner et/ou radiographiques et/ou échographies et/ou IRM du fémur B sont utilisées. Dans tous les cas, à l'issue de cette étape préalable d'acquisition des données, on dispose de suffisamment d'informations pour concevoir et fabriquer un bloc fémoral 10 spécifique au patient. Le bloc 10 est montré, d'une part, en position sur le fémur B à la figure 1 et, d'autre part, seul aux figures 2à5.

Plus précisément, le bloc fémoral spécifique 10 présente une surface 12 prévue pour être tournée vers le fémur B, et une surface 14 prévue pour être tournée vers le chirurgien lorsque le bloc 10 est ajusté sur le fémur B. La surface 12 est conformée de manière spécifiquement ajustée à l'extrémité inférieure du fémur B et, en service, est appuyée fixement contre cette extrémité du fémur B, en épousant la surface de cette dernière par complémentarité de formes. On comprend que, pour aboutir à un tel ajustement rigoureux entre la surface d'appui 12 et l'extrémité inférieure du fémur B, la surface 12 est dessinée en utilisant les données de cartographie pré-opératoires relatives au fémur. De la sorte, la surface d'appui 12 présente des reliefs personnalisés spécifiquement au patient opéré qui, en coopérant avec des reliefs complémentaires délimités par la surface de l'extrémité inférieure du fémur, n'autorisent qu'une seule configuration d'appui ajusté sur le fémur B, comme représenté sur la figure 1. A titre d'exemple, dans le mode de réalisation considéré sur cette figure 1, la surface d'appui 12 recouvre des zones antérieure et distale de l'extrémité inférieure du fémur B, en épousant de manière ajustée les reliefs de ces zones.

En service, le bloc fémoral spécifique 10 est destiné à être appuyé fixement, par sa surface 12, sur l'extrémité inférieure du fémur B. Dans l'exemple de réalisation considéré, cette fixation est réalisée grâce à, d'une part, deux trous traversants 27 et 28 ménagés à travers deux plots de guidage, respectivement 25 et 26, ces trous 27 et 28 reliant la surface 12 avec la surface 14 opposée du bloc 10 et, d'autre part, deux broches d'ancrage osseux 61 et 62 à même d'être respectivement engagées de façon complémentaire dans les trous traversants 27 et 28, comme représenté sur la figure 1, jusqu'à se planter et ainsi s'immobiliser dans la matière osseuse du fémur B.

A ce stade, on remarque que le bloc fémoral spécifique 10 est constitué de deux parties ayant des finalités respectives différentes, comme expliqué progressivement ci-après, à savoir une partie principale 20, ainsi qu'une partie secondaire 30 qui est détachable de la partie principale 20. Une zone de jonction 16 relie les parties 20 et 30 du bloc fémoral spécifique 10, qui se présente sous une forme initialement monolithique. Le bloc 10 peut être fabriqué par tout moyen connu, adapté à la présente application. De préférence, le bloc 10 peut être fabriqué par frittage laser sélectif de poudre (« selective laser sintering » en anglais). Dans ce cas, le bloc 10 solidifié est entouré de poudre résiduelle après fabrication, puis la poudre est enlevée d'abord par balayage puis par soufflage ; le bloc 10 peut alors être retiré du bac de fabrication, nettoyé puis stérilisé avant d'être utilisé sur un patient.

Comme montré à la figure 1, la partie principale 20 comprend deux branches 21 et 22 qui s'étendent depuis une portion centrale 23. Les plots 25 et 26 sont formés sur la surface 14 du bloc 10, respectivement sur les branches 21 et 22. Autrement dit, la branche 21 est traversée par le trou 27, tandis que la branche 22 est traversée par le trou 28. La portion centrale 23 est munie, en saillie depuis la surface 14, d'un plot 29 de fixation d'un bloc de coupe ou de tout autre système de préparation du fémur B, non représentés, lors d'une étape per-opératoire ultérieure. La portion centrale 23 délimite à elle seule la majeure partie de la matière du bloc 10 et de la surface d'appui 12.

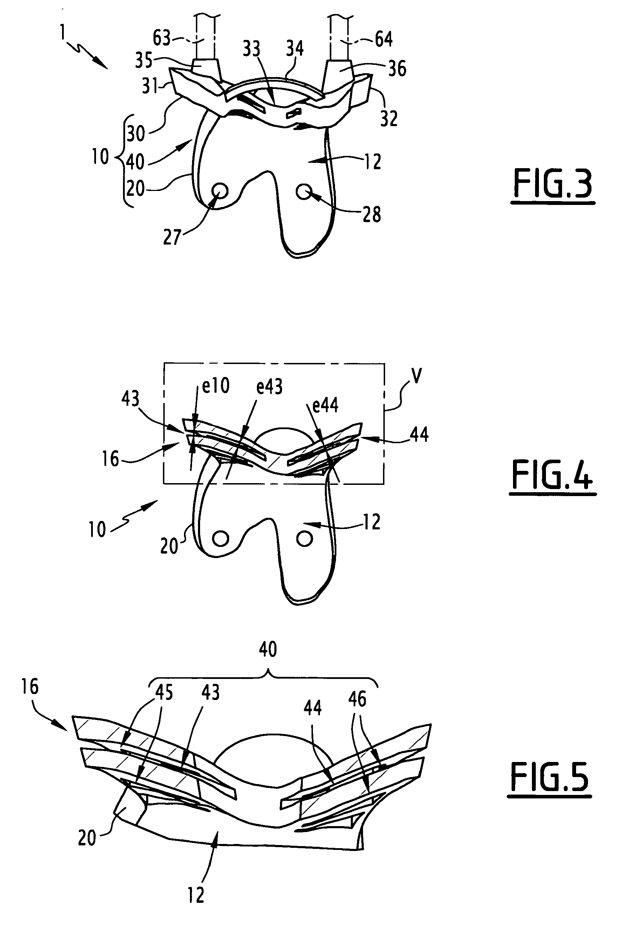

Dans le mode de réalisation des figures 1 à 5, la partie secondaire 30 comprend deux branches 31 et 32 qui s'étendent depuis la portion centrale 23 au niveau de la zone de jonction 16, dans le prolongement des branches 21 et 22 respectivement, en suivant la surface de du fémur B. Les éléments 21, 22, 23, 31 et 32 délimitent ensemble la surface d'appui 12. Des plots 35 et 36 sont formés sur la surface 14 du bloc 10, respectivement sur les branches 31 et 32. La partie secondaire 30 est pourvue de deux trous traversants 37 et 38 ménagés dans les plots de guidage, respectivement 35 et 36, qui sont chacun prévus pour recevoir un outil de préparation osseuse, tel qu'un foret de perçage ou une broche d'ancrage osseux : sur la figure 3, de tels outils de préparation osseuse, respectivement référencés 63 et 64, sont montrés en pointillés. Les branches 31 et 32 sont séparées par un espace intermédiaire 33, destiné à être placé en service dans le prolongement de l'espace intercondylaire, comme montré à la figure 1. Une plaque 34 s'étend à travers l'espace 33 en reliant les branches 31 et 32, notamment dans un but de rigidification de la partie secondaire 30. Cette plaque 34 ne définit pas la surface d'appui 12 et est donc, en service, distante de la surface du fémur B. Ainsi, la plaque 34 n'entre pas dans l'espace intercondylaire et ne le cache pas intégralement. En alternative, la plaque 34 peut comporter des inscriptions utiles au chirurgien, ou le bloc 10 ne comporte pas de plaque 34.

En service, la partie principale 20 est présente pendant toute l'utilisation de l'instrumentation 1, dans le sens où cette partie principale 20 est, à la fois, la première mise en place, en étant fixée au fémur B par les broches 61 et 62 que l'on peut ainsi qualifier de broches primaires, et la dernière dégagée vis-à-vis du fémur, tandis que la partie secondaire 30 est, au contraire, conçue pour, au cours de l'utilisation de l'instrumentation, pouvoir être séparée de la partie principale 20 par le chirurgien. Dans la mesure où le bloc fémoral spécifique 10 est mis à disposition du chirurgien sous une forme initialement monolithique, on comprend que la séparation de la partie secondaire 30 vis-à-vis de la partie principale 20 nécessite pour le chirurgien de rompre le lien de matière entre les parties 20 et 30, au niveau de la zone de jonction 16. En fait, la présence temporaire, au cours de l'intervention, de la partie secondaire 30 vis-à-vis du reste du bloc 10 permet de bien comprendre l'intérêt de la présente invention.

En effet, la partie secondaire 30 correspond à la partie du bloc 10 qui a été conçue, de manière pré-opératoire, pour guider l'application per-opératoire sur le fémur B des outils de préparation 63 et 64, tels qu'un foret de perçage ou une broche d'ancrage, via les trous 37 et 38. Dans le détail de la pratique chirurgicale, on comprend que deux approches opératoires sont possibles en ce qui concerne les trous 37 et 38 :
- soit ces trous 37 et 38 servent à guider l'application d'un foret de perçage ou d'un outil similaire, qui est dégagé dès que son action de perçage du fémur est terminée ; dans ce cas, à une étape ultérieure de la chirurgie, les trous borgnes, qui ont été ainsi percés dans le fémur, sont utilisés pour recevoir des broches d'ancrage secondaire dont la finalité est expliquée un peu plus bas ;
- soit ces trous 37 et 38 servent à guider l'application de broches d'ancrage ou similaires, ces broches étant éventuellement mises en place après qu'un pré-trou a été réalisé dans le fémur par un outil de perçage introduit à travers les trous 37 et 38 ; dans ce cas, une fois que ces broches d'ancrage sont mises en place via les trous 37 et 38, elles demeurent avantageusement en place jusqu'à l'étape ultérieure précitée et constituent ainsi des broches d'ancrage secondaires, similaires à celles définies juste ci-dessus.

Dans tous les cas, on note que, lors de la conception sur mesure du bloc 10, les trous 37 et 38 ont été positionnés au sein du bloc 10 de manière à orienter précisément les broches secondaires précitées : au cours de la chirurgie, lors de l'étape ultérieure précitée, ces broches secondaires sont utilisées pour, à leur tour, guider le positionnement de moyens de coupe du fémur B, tels qu'un bloc de coupe, afin de réséquer l'extrémité du fémur B selon un ou plusieurs plans de résection dimensionnés pour former ultérieurement des appuis plans correspondants pour la fixation d'un composant prothétique fémoral. Ainsi, le dégagement de la partie secondaire 30 permet de libérer de l'espace en regard de la zone du fémur B sur laquelle débouchaient jusqu'alors les trous 37 et 38, à savoir la zone distale de l'extrémité inférieure du fémur B.

A cet effet, l'instrumentation 1 comprend des moyens 40 de dégagement mécanique de la partie secondaire 30 par rapport à la partie principale 20 et au fémur B, ces moyens 40 étant disposés dans la zone de jonction 16 entre la partie principale 20 et la partie secondaire 30. Sur l'exemple des figures 1 à 5, les moyens de dégagement mécanique 40 sont venus de matière à la fois avec la partie principale 20 et la partie secondaire 30, notamment lors de la fabrication du bloc 10 par frittage laser de poudre.

Etant donné l'agencement des branches 31 et 32 avec la portion centrale 23, les moyens 40 sont répartis entre une première région 41 située entre la branche 31 et la portion 23 et une deuxième région 42 située entre la branche 32 et la portion 23. Dans chaque région 41 et 42, les moyens 40 comprennent une cavité, respectivement 43 et 44. Chaque cavité 43 et 44 traverse le bloc 10 dans la zone de jonction 16 et débouche de part et d'autre des branches 31 et 32, au niveau de l'espace 33 et à l'opposé de cet espace 33, c'est-à-dire globalement suivant la direction latéro-médiale en service. Chaque cavité 43 et 44 est dite enveloppante, dans la mesure où elle s'étend à travers le bloc 10 en épousant la forme extérieure du fémur B, autrement dit en suivant la géométrie de la surface 12, et peut donc être qualifiée de cavité spécifique au patient, correspondant à sa morphologie particulière. Comme montré aux figures 2 et 4, le bloc 10 présente une épaisseur e10, mesurée entre la surface 12 et la surface 14 dans une direction normale à la surface 12, qui est sensiblement constante dans la zone de jonction 16 où sont situés les moyens 40. En outre, chaque cavité 43 et 44 présente une épaisseur, respectivement e43 et e44, qui est constante dans la direction normale à la surface 12.

Comme montré à la figure 1, les moyens 40 comprennent également des ouïes 45 et 46, ménagées à travers le bloc 10, respectivement dans la région 41 et la région 42. Les ouïes 45 débouchent dans la cavité 43, avec deux ouïes 45 qui débouchent au niveau de la surface 12 et deux ouïes 45 qui débouchent au niveau de la surface 14. De même, les ouïes 46 débouchent dans la cavité 44, avec deux ouïes 46 qui débouchent au niveau de la surface 12 et deux ouïes 46 qui débouchent au niveau de la surface 14. Les ouïes 45 et 46 présentent un profil adapté pour faciliter la déformation de la matière dans chacune des régions 41 et 42. Sur l'exemple non limitatif des figures 1 à 5, les ouïes 45 et 46 présentent une forme de quadrilatère irrégulier, avec notamment une face plane et une face de forme courbe sensiblement hyperbolique placées en vis-à-vis, qui s'étendent suivant la direction normale à la surface 12.

Lorsque le bloc 10 est fabriqué par frittage de poudre, les cavités 43 et 44 débouchent hors du bloc 10, sur les côtés des branches 31 et 32 et au niveau des ouïes 45 et 46, ce qui permet l'évacuation de poudre. Dans le cas contraire, la poudre emprisonnée dans les cavités risquerait de se déverser sur l'os du patient. Autrement dit, dans le cadre de l'invention, les cavités 43 et 44 comportent au moins une ouverture débouchant hors du bloc 10.

En pratique, le dégagement mécanique de la partie secondaire 30 par rapport à la partie principale 20 et au fémur B, sur lequel est fixée cette partie principale 20 par les broches primaires 61 et 62, est réalisé par rupture de la matière du bloc 10 au niveau des moyens 40. Les régions 41 et 42 peuvent être pliées et arrachées lorsque la partie 30 est écartée du fémur B, avec les orifices 37 et 38. En effet, les cavités 43 et 44 et les ouïes 45 et 46 constituent des amorces de rupture facilitant la séparation des parties 20 et 30. Autrement dit, les moyens 40 constituent des éléments d'affaiblissement localisé de la matière du bloc 10 dans la zone 16. Le dégagement mécanique peut être effectué manuellement ou, de préférence, à l'aide d'un outil, notamment du type ostéotome, tournevis ou écarteur. Optionnellement, cet outil de dégagement peut être conformé pour pouvoir se loger en même temps dans les ouïes 45 et 46, de sorte qu'une action du chirurgien sur l'outil puisse arracher ou déformer la matière des régions 41 et 42 simultanément.

Grâce aux moyens 40 intégrés directement au bloc 10, ce bloc 10 est donc adapté pour être dégagé du fémur B, au niveau de la partie secondaire 30, avec les broches 61 et 62 qui sont en place dans le bloc 10 et le fémur B. On comprend que, selon le détail de la procédure chirurgicale suivie en per-opératoire, ce dégagement de la partie secondaire 30 peut être réalisé soit alors que les trous 37 et 38 sont libres, après qu'ils ont guidé un foret de perçage ou similaire, soit alors que des broches secondaires ont été passées dans ces trous et demeurent en place dans le fémur jusqu'à l'étape opératoire subséquente inclusivement, comme expliqué plus haut. Dans le second cas, on notera que le dégagement de la partie secondaire 30 est accompagné du coulissement de cette partie 30 le long des broches secondaires précitées. Bien entendu, dans le premier cas, le dégagement de la partie secondaire 30 peut être effectué dans d'autres directions, en s'éloignant du fémur B, étant cependant remarqué que, dans tous les cas, la séparation de la partie secondaire 30 vis-à-vis de la partie principale 20 nécessite de pouvoir écarter la partie 30 dans une direction non bloquante entre cette partie 30 et le fémur, alors que, eu égard à l'enveloppement ajusté du fémur par la surface 12 du bloc 10, la partie 30 est coincée dans de nombreuses directions vis-à-vis des reliefs spécifiques du fémur, épousés par cette partie 30.

A l'issue de cette étape chirurgicale, la partie 30 a été dégagée et le fémur porte les broches secondaires précitées, étant rappelé que ces dernières sont soit celles qui ont été passées dans les trous 37 et 38, en tant qu'outils de préparation osseuse 63 et 64, et qui ont été laissées en place dans le fémur, soit des broches qui, après que la partie 30 a été dégagée, sont rapportées et ancrées dans les trous borgnes réalisés juste avant dans le fémur par un foret de perçage ou similaire, qui a agi sur le fémur à travers les trous 37 et 38, en tant qu'outil de préparation osseuse 63 et 64.

Une fois que le chirurgien a dégagé la partie secondaire 30 du bloc 10 en l'écartant de la surface du fémur B, il poursuit l'intervention chirurgicale. Le chirurgien utilise un bloc de coupe ou, plus généralement, tout système de préparation du fémur, en positionnant ce bloc ou ce système à l'aide des broches secondaires précitées.

En variante non représentée, le bloc 10 peut être conformé différemment, notamment pour s'adapter à un os différent du fémur B de la figure 1. Ainsi, la partie secondaire 30 peut ne pas comporter pas deux branches 31 et 32, ou bien comporter une portion intermédiaire agencée entre la portion 23 et les branches 31 et 32. Dans ce cas, les moyens de dégagement 40 peuvent être répartis dans une seule région de dégagement située entre cette portion intermédiaire et la portion 23. Une telle région de dégagement est alors conformée de manière comparable à l'une des régions 41 ou 42 décrites plus haut, avec une seule cavité enveloppante et des ouïes dont les profils respectifs sont adaptés à cet agencement particulier. Dans le prolongement des considérations qui précèdent, on comprend que la présence, au sein de la partie 30, des branches 31 et 32 décrites ci-dessus ou, plus généralement, la présence de deux ou davantage de branches similaires, plus ou moins individualisées, n'est pas limitative de l'invention.

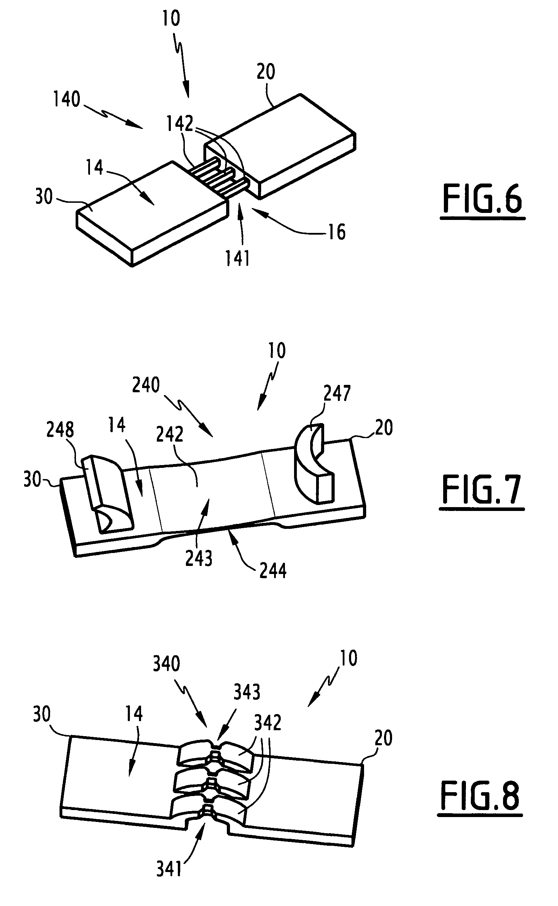

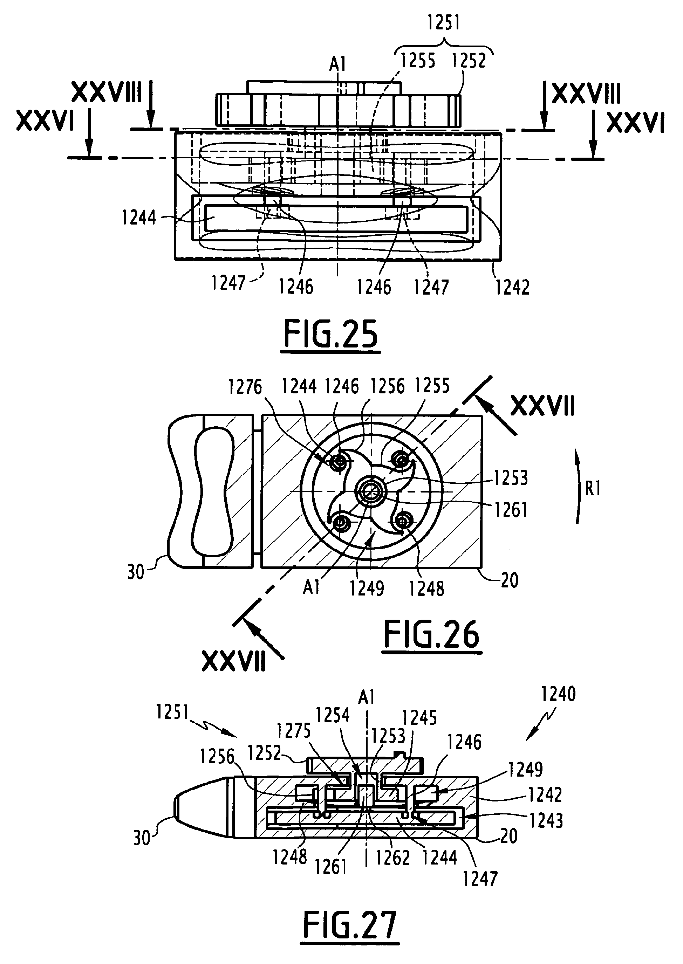

Sur les figures 6 à 28 sont représentées différentes variantes de moyens de dégagement mécanique adaptés pour équiper l'instrumentation 1.

Quel que soit le mode de réalisation, l'instrumentation 1 comprend un bloc spécifique 10 muni de moyens de dégagement mécanique de la partie secondaire 30 par rapport à la partie principale 20. Le bloc 10 et ses parties 20 et 30 sont représentés partiellement et schématiquement aux figures 6 à 28, au niveau de la zone de jonction 16. Dans un but de simplification, les parties 20 et 30 sont représentées par des éléments parallélépipédiques alignés l'un par rapport à l'autre, avec une seule région de dégagement entre elles. La surface 14 est référencée uniquement pour illustrer l'orientation du bloc 10 et des moyens de dégagement mécanique par rapport à l'os B, non représenté, tandis que la surface d'appui n'est pas référencée. En regard de cette représentation schématique, on comprend néanmoins que le bloc 10 est conformé pour épouser la surface de l'os B, comme sur l'exemple des figures 1 à 5.

Sur la figure 6 sont représentés des moyens de dégagement mécanique 140 appartenant à un deuxième mode de réalisation d'une instrumentation 1.

Un espace intermédiaire 141 est ménagé entre les parties 20 et 30, formant ainsi une discontinuité de la surface 14. Les moyens 140 comprennent des barres 142, plus précisément trois barres 142 sur l'exemple de la figure 6, qui s'étendent à travers l'espace intermédiaire 141 et relient mécaniquement les parties 20 et 30. Ces barres 142 sont facilement destructibles par cassure ou arrachement, lorsqu'une tension suffisante est exercée par le chirurgien sur la partie secondaire 30, selon une direction d'éloignement par rapport à la partie principale 20. Si la matière des barres 142 est plutôt fragile, celles-ci cassent brutalement, alors que si la matière des barres 142 est plutôt ductile, celles-ci vont se déformer par constriction, notamment jusqu'à séparation des parties 20 et 30. Ainsi, le dégagement mécanique de la partie 30 par rapport à la partie 20 et à l'os B est réalisé, de manière irréversible, par rupture des barres 142. En alternative, les moyens 140 peuvent comprendre un nombre différent de barres 142, ou celles-ci peuvent être conformées différemment.

Sur la figure 7 sont représentés des moyens de dégagement mécanique 240 appartenant à un troisième mode de réalisation d'une instrumentation 1.

Les moyens 240 comprennent une plaquette 242 sensiblement plane qui relie mécaniquement les parties 20 et 30. La plaquette 242 est plus mince que les parties 20 et 30, avec une première cuvette concave 243 ménagée sur la plaquette 242 du côté de la surface 14 et une deuxième cuvette concave 244 ménagée sur la plaquette 242 du côté opposé à la surface 14. Sur l'exemple de la figure 7, la cuvette 244 forme un évidement de matière plus prononcé que la cuvette 243. Les moyens 240 comprennent également des poignées de manipulation 247 et 248 ménagées sur la surface 14, respectivement sur la partie principale 20 et sur la partie secondaire 30. Sur l'exemple de la figure 7, la poignée 247 est conformée pour recevoir l'index du chirurgien, tandis que la poignée 248 est conformée pour recevoir le pouce du chirurgien. En pratique, le dégagement mécanique de la partie 30 par rapport à la partie 20 et à l'os B est réalisé, de manière réversible, simplement par pliage de la plaquette 242. Comme le pliage permet un dégagement réversible de la partie 30, le chirurgien a la possibilité de repositionner cette partie secondaire 30 sur l'os B pour réaliser une reprise des perçages, si besoin. En alternative, la plaquette 242 et les cuvettes 243 et 244, de même que les poignées 247 et 248, peuvent être conformées différemment.

Sur la figure 8 sont représentés des moyens de dégagement mécanique 340 appartenant à un quatrième mode de réalisation d'une instrumentation 1.

Un espace intermédiaire 341 est ménagé entre les parties 20 et 30, formant ainsi une discontinuité de la surface 14. Les moyens 340 comprennent des arches 342, plus précisément trois arches 342 sur l'exemple de la figure 8, qui s'étendent par dessus l'espace intermédiaire 341, en étant courbées en s'éloignant de la surface 14, et relient mécaniquement les parties 20 et 30. Chaque arche 342 comporte une portion 343 amincie par rapport au reste de l'arche 342. Les portions amincies 343 sont situées à mi-chemin des parties 20 et 30 et sont alignées suivant un même axe. Ces arches 342 sont facilement destructibles sous tension ou, de manière encore plus pratique pour le chirurgien, par section à l'aide d'un outil de type pince coupante. Le dégagement mécanique irréversible de la partie 30 par rapport à la partie 20 et à l'os B est donc réalisé, de préférence, par section des arches 342 au niveau des portions 343. En alternative, les moyens 340 peuvent comprendre un nombre différent d'arches 342, ou celles-ci peuvent être conformées différemment.

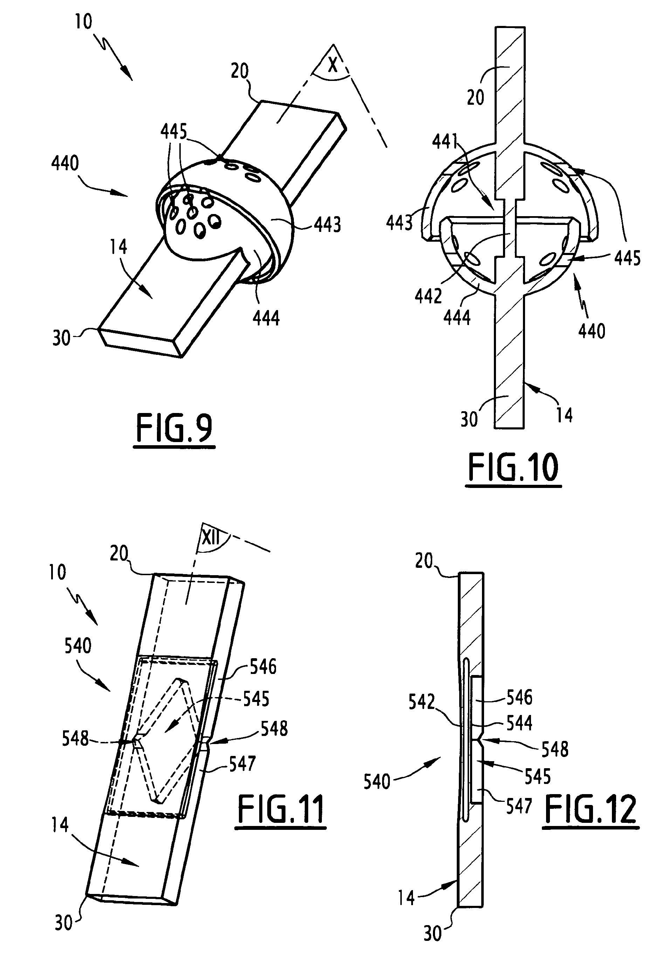

Sur les figures 9 et 10 sont représentés des moyens de dégagement mécanique 440 appartenant à un cinquième mode de réalisation d'une instrumentation 1.

Les moyens 440 comportent un espace 441 et des barres 442, analogues respectivement à l'espace 141 et aux barres 142 des moyens 140 décrits plus haut. Les moyens 440 comprennent également un premier dôme hémisphérique 443 ménagé sur la partie 20 et un deuxième dôme hémisphérique 444 ménagé sur la partie 20. Le dôme 443 présente un diamètre supérieur au diamètre du dôme 444, de sorte que le dôme 444 est en partie logé à l'intérieur du dôme 443, sans contact entre eux. Ensemble, les deux dômes 443 et 444 entourent l'espace 441 et les barres 442. En outre, une pluralité d'orifices traversants 445 sont ménagés dans chacun des dômes 443 et 444. Ces dômes sont prévus pour récupérer d'éventuels débris dans le cas d'une rupture fragile des barres 442. En effet, de tels débris risqueraient de s'introduire dans le corps du patient, ce qu'il faut impérativement éviter en chirurgie. Les orifices 445, ainsi que l'écart entre les dômes 443 et 444, sont dimensionnés pour éviter l'échappement de tels débris, tout en permettant le soufflage de poudre contenue dans les dômes 443 et 444 après fabrication du bloc 10 et des moyens 40 par frittage. Lors de la rupture des barres 442, les dômes 443 et 444 sont en liaison pivot puis le dôme 444 peut être dégagé hors du dôme 443, en veillant à évacuer d'éventuels débris.

Dans ce mode de réalisation, on comprend que les parties 20 et 30 sont munies, au niveau de la zone de jonction 16, de portions de matière additionnelle permettant d'écarter les moyens 440 par rapport à la surface d'appui du bloc 10 et à l'os B. En effet, la présence des dômes 443 et 444 empêche de positionner les moyens 440 à proximité de l'os B.

Sur les figures 11 et 12 sont représentés des moyens de dégagement mécanique 540 appartenant à un sixième mode de réalisation d'une instrumentation 1.

Les moyens 540 comprennent une plaquette pliable 542 comparable à la plaquette 242 décrite plus haut, ainsi qu'une plaquette destructible 544. Les moyens 540 comprennent également une cavité 545 en forme de losange, débouchante à l'opposé de la face 14 et fermée au niveau de la plaquette 544. La cavité en losange 545 présente deux sommets dirigés respectivement vers les parties 20 et 30, ainsi que deux sommets comportant chacun une zone 548 d'affaiblissement localisé de la matière entre une portion 546 solidaire de la partie 20 et une portion 547 solidaire de la partie 30. Lorsque le chirurgien manipule la partie 30 selon une action de pliage de la plaquette 542, de manière comparable au pliage de la plaquette 242, alors les zones 548 cèdent et leur rupture, en se propageant en direction de la surface 14, entraine une déchirure de la plaquette 544 et une séparation des portions 546 et 547. Ainsi, le dégagement mécanique de la partie 30 par rapport à la partie 20 et à l'os B est réalisé en combinant pliage et rupture. Ce dégagement est réversible grâce à la présence de la plaquette 542. En alternative, les moyens 540 combinant pliage et rupture peuvent être conformés de manière différente.

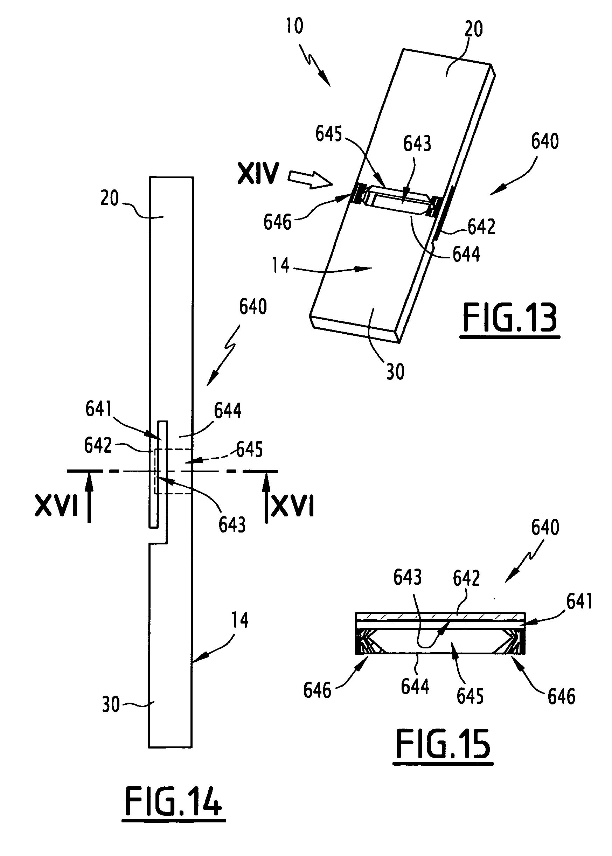

Sur les figures 13 à 15 sont représentés des moyens de dégagement mécanique 640 appartenant à un septième mode de réalisation d'une instrumentation 1.

Les moyens 640 comprennent une languette 642 et une portion 644 séparées par un espace intermédiaire 641. La languette 642 est située du côté opposé à la surface 14 et raccordée à la partie 20, mais pas à la partie 30. La portion 644 est située du côté de la surface 14 et raccordée à la fois à la partie 20 et à la partie 30. De préférence, l'épaisseur de la portion 644, mesurée dans une direction normale à la surface 14, est supérieure à l'épaisseur de la languette 642. La portion 644 comporte une cavité 645 de réception d'un outil, non représenté, de dégagement mécanique de la partie 30. Cette cavité 645 débouche dans l'espace 641 et au niveau de la surface 14. De part et d'autre de la cavité 645, la portion 644 comprend des éléments filamentaires 646 reliant le côté raccordé à la partie 20 au côté raccordé à la partie 30. La languette 642 comporte, en regard de la cavité 645 suivant la direction normale à la surface 14, un logement 643 d'appui de l'outil introduit à travers la cavité 645. De préférence, cet outil est du type écarteur, présentant initialement une forme adaptée pour pénétrer dans la cavité 645.

En pratique, dans un premier temps, l'outil est introduit dans la cavité 645 puis vient en butée contre la languette 642, dans le logement 643. Dans un deuxième temps, l'outil est déployé dans la cavité 645, de manière à exercer des efforts opposés sur les éléments 646, jusqu'à leur déchirement entraînant la séparation irréversible des parties 20 et 30. Les moyens 640, en particulier les éléments 646, peuvent être conformés différemment sans sortir du cadre de l'invention, en formant un lien de matière entre les parties 20 et 30, tout en étant facilement destructibles sous l'action de l'outil introduit dans la cavité 645. La présence de la languette 642 évite que l'outil vienne racler contre l'os B. Le logement 643 est optionnel, mais facilite le positionnement de l'outil au sein des moyens 640.

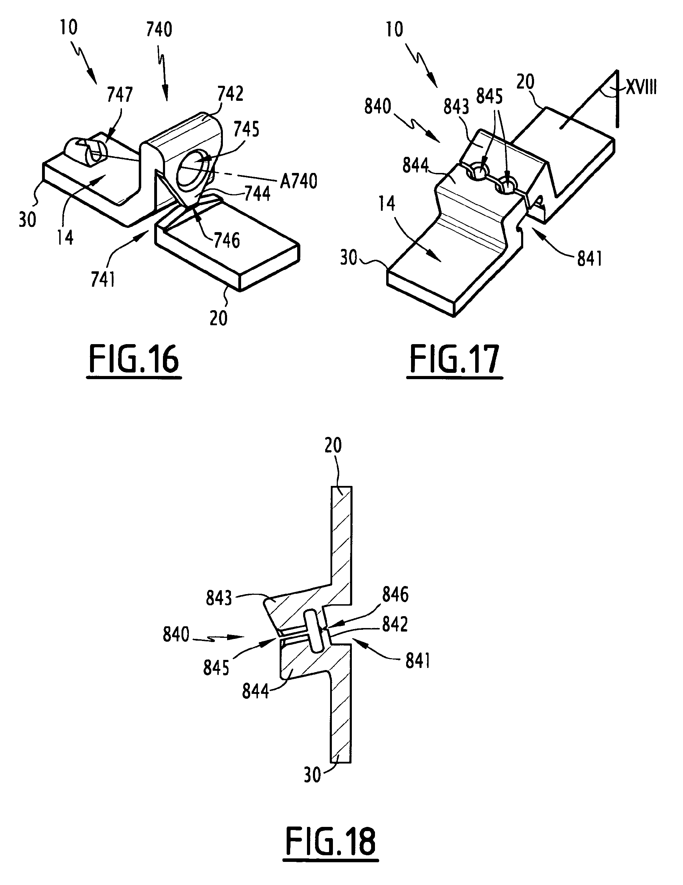

Sur la figure 16 sont représentés des moyens de dégagement mécanique 740 appartenant à un huitième mode de réalisation d'une instrumentation 1.

Un espace intermédiaire 741 est ménagé entre les parties 20 et 30. Les moyens 740 comprennent un organe 742 qui s'étend en saillie de la surface 14, par-dessus l'espace 741. L'organe 742 comporte une languette 744 rattachée à la partie 20 par une portion amincie 746, qui forme ainsi une zone d'affaiblissement localisé de la matière. L'organe 742 est traversé par un orifice 745 centré sur un axe A740 et prévu pour recevoir un outil, par exemple du type tournevis. De préférence, un élément de guidage 747 est ménagé sur la surface 14 de la partie 30, dans le prolongement de l'axe A740, pour recevoir la pointe de l'outil. En pratique, l'outil est introduit dans l'orifice 745 suivant l'axe A740 dans un premier temps, puis est relevé en prenant appui dans l'élément 747 et en exerçant des efforts opposés à la surface 14 à l'intérieur de l'orifice 745 dans un deuxième temps, jusqu'à ce que le dégagement mécanique irréversible de la partie 30 se produire par rupture de la portion 746 dans un troisième temps. En alternative, l'organe 742, l'orifice 745 et la portion 746 peuvent être conformés différemment sans sortir du cadre de l'invention. Par exemple, la portion amincie 746 peut relier l'organe 742 à la partie 30, ou bien l'organe 746 peut comporter deux portions amincies le reliant à chacune des parties 20 et 30. Selon un autre exemple, l'orifice 745 peut présenter une forme différente de l'exemple de la figure 16, par exemple une forme carrée permettant la rupture de la portion 746 par rotation de l'outil dans l'orifice 745.

Sur les figures 17 et 18 sont représentés des moyens de dégagement mécanique 840 appartenant à un neuvième mode de réalisation d'une instrumentation 1.

Un espace intermédiaire 841 est ménagé entre les parties 20 et 30. Les moyens 840 comprennent une plaquette 842 qui relie un premier élément 843 solidaire de la partie 20 avec un deuxième élément 844 solidaire de la partie 30. Les éléments 843 et 844 sont formés en saillie au-delà de la surface 14, tandis que la plaquette 842 s'étend par-dessus l'espace 841. La plaquette 842 comporte une portion amincie 846, formant ainsi une zone d'affaiblissement localisé de la matière. Deux trous 845 sont ménagés entre les éléments 843 et 844, en regard de la portion 846. Les trous 845 sont prévus pour l'introduction et le positionnement d'un outil, susceptible de venir appuyer contre la portion 846 jusqu'à sa rupture. En alternative, les moyens 840 peuvent être conformés différemment sans sortir du cadre de l'invention, par exemple avec un nombre de trous 845 qui dépend de la largeur du bloc 10 au niveau de la zone de jonction 16.

Sur les exemples des figures 1 à 18, les moyens de dégagement mécanique 40 à 840 sont de préférence venus de matière à la fois avec les parties 20 et 30 du bloc 10.

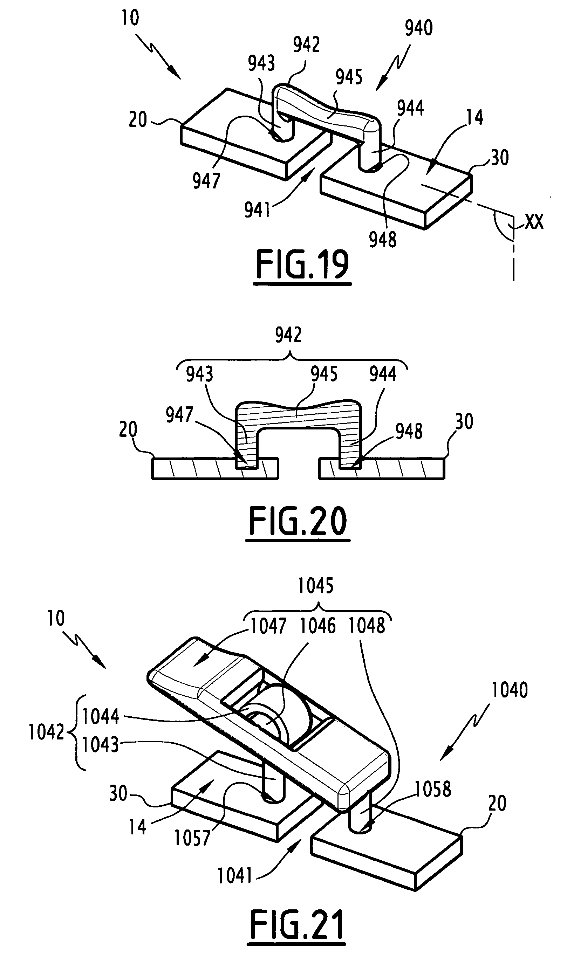

Sur les figures 19 et 20 sont représentés des moyens de dégagement mécanique 940 appartenant à un dixième mode de réalisation d'une instrumentation 1.

Un espace intermédiaire 941 est ménagé entre les parties 20 et 30. Les moyens 940 comprennent un organe 942 en forme de pont ou de U, avec deux pattes 943 et 944 sont reliés par une portion centrale 945. Des orifices 947 et 948 sont ménagés respectivement dans les parties 20 et 30, en débouchant au niveau de la surface 14. En pratique, l'organe 942 est solidarisé aux parties 20 et 30 durant une phase pré-opératoire, avec la patte 943 qui est clipsée dans l'orifice 947, la patte 944 qui est clipsée dans l'orifice 948, tandis que la portion 945 s'étend par-dessus l'espace 941. Durant une phase per-opératoire, l'organe 942 est amovible du bloc spécifique 10 par déclipsage des pattes 943 et 944. Autrement dit, la liaison entre l'organe 942 et les parties 20 et 30 est temporaire, et le dégagement mécanique de la partie 30 est réversible. Ainsi, cette partie 30 peut être facilement repositionnée sur l'os si besoin.

Sur la figure 21 sont représentés des moyens de dégagement mécanique 1040 appartenant à un onzième mode de réalisation d'une instrumentation 1.

Un espace intermédiaire 1041 est ménagé entre les parties 20 et 30. Les moyens 1040 comprennent un organe 1042 muni d'une tige 1043 solidarisée à la partie 30 et d'un palier 1044 positionné à l'extrémité de la tige 1043 opposée à la partie 30. De préférence, la tige 1043 est vissée ou clipsée dans un orifice 1057 ménagé dans la partie 30, au niveau de la surface 14. En alternative, la tige 1043 est venue de matière avec la partie 30. Les moyens 1040 comprennent également un organe 1045 de forme globalement parallélépipédique, muni d'une portion centrale 1046 en liaison pivot dans le palier 1044, d'une première extrémité d'appui 1047 et d'une deuxième extrémité, opposée à l'extrémité 1047 par rapport à la portion centrale 1046, comportant une tige 1048 qui s'étend en direction de la surface 14 de la partie 20. Un orifice 1058 est ménagé dans la partie 20, au niveau de la surface 14. Les moyens 1040 sont solidarisés aux parties 20 et 30 durant une phase pré-opératoire, avec la tige 1048 qui est clipsée dans l'orifice 1058 et l'organe 1045 qui s'étend par-dessus l'espace 1041. Durant une phase per-opératoire, un simple appuie du chirurgien sur l'extrémité 1047 permet, par effet de levier, de faire pivoter l'organe 1045 dans le palier 1044 et de déclipser la tige 1048 de l'orifice 1058. Autrement dit, la liaison entre les moyens 1040 et les parties 20 et 30 est temporaire, et le dégagement mécanique réversible de la partie 30 peut être effectué par simple déclipsage. Ainsi, cette partie 30 peut être facilement repositionnée sur l'os si besoin.

Sur les figures 19 à 21, les moyens 940 et 1040 peuvent être en partie fabriqués en matériau élastique. A titre d'exemple non limitatif, les éléments 942, 1043 et 1048 peuvent être fabriqués en matériau élastique. Un tel matériau élastique, d'une part, contribue à la mise sous tension de la partie principale et la partie secondaire et, d'autre part, garantit la résistance mécanique de l'ensemble lors du positionnement du bloc, ainsi que lors du perçage.

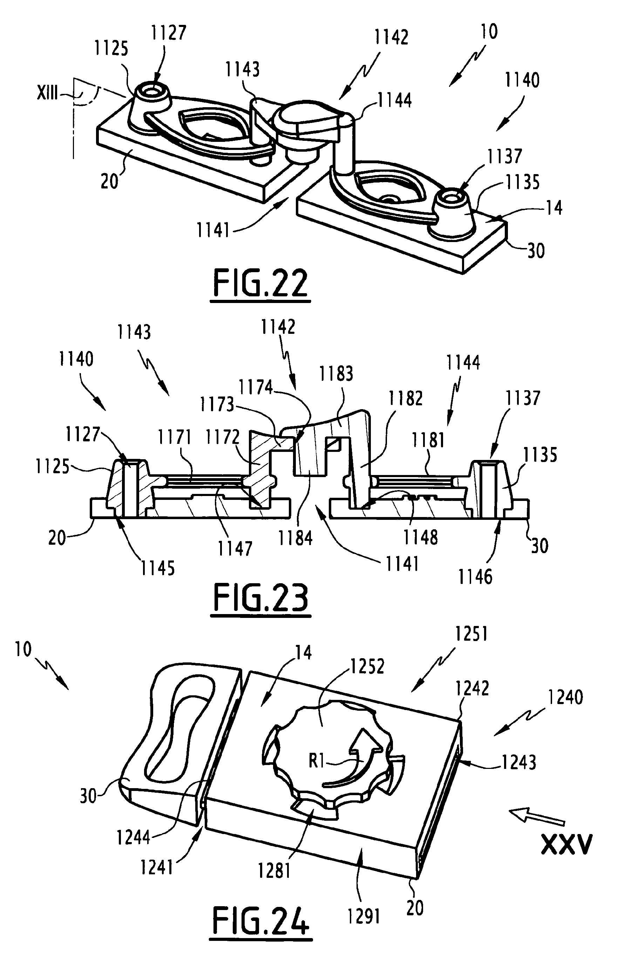

Sur les figures 22 et 23 sont représentés des moyens de dégagement mécanique 1140 appartenant à un douzième mode de réalisation d'une instrumentation 1.

Un espace intermédiaire 1141 est ménagé entre les parties 20 et 30. Les moyens 1140 comprennent un premier organe de liaison 1143 et un deuxième organe de liaison 1144 adaptés pour former une liaison mécanique 1142 du type pont entre les parties 20 et 30, par-dessus l'espace 1141. L'organe 1143 comprend un plot 1125 muni d'un trou traversant 1127, de deux branches 1171 reliant le plot 1125 à une patte 1172, d'une portion 1173 positionnée à une extrémité de la patte 1172 opposée aux branches 1171 et d'un orifice 1174 ménagé sur la portion 1173. L'organe 1144 comprend un plot 1135 muni d'un trou traversant 1137, de deux branches 1181 reliant le plot 1135 à une patte 1182, d'une portion 1183 positionnée à une extrémité de la patte 1182 opposée aux branches 1181 et d'un pion 1184 ménagé sur la portion 1183 parallèlement à la patte 1182. Des orifices 1145 et 1147 sont ménagés dans la partie 20, tandis que des orifices 1146 et 1148 sont ménagés dans la partie 30, débouchant au niveau de la surface 14. Les plots 1125 et 1135 et les trous 1127 et 1137 sont comparables respectivement aux plots 25 et 35 munis d'orifices 27 et 37 du premier mode de réalisation, décrit plus haut, autrement dit sont conformés pour le guidage d'outil ou de broches. Les pattes 1172 et 1182 sont comparables aux pattes 943 et 944 du dixième mode de réalisation, décrit plus haut. Les branches 1171 et 1181 sont conformés pour suivre le profil de la surface 14 entre les plots et les pattes. Les portions 1173 et 1183 sont conformés pour venir en appui l'une contre l'autre, avec le pion 1184 qui vient se loger dans l'orifice 1174, formant ainsi la liaison 1142. Les éléments constitutifs des organes 1143 et 1144 peuvent être conformés de manière différente.

En pratique, les moyens 1140 sont solidarisés aux parties 20 et 30 durant une phase pré-opératoire, avec les éléments 1125, 1135, 1172 et 1182 qui sont clipsés respectivement dans les orifices 1145, 1146, 1147 et 1148, tandis que la liaison 1142 est formée par-dessus l'espace 1141. Plus précisément, l'organe 1143 est fixé à la partie 20, puis l'organe 1144 est fixé à la partie 30 et à l'organe 1143. De préférence, la mise en oeuvre de canons de perçage métallique permet de réaliser l'ensemble des orifices 1145, 1146, 1147 et 1148 avec une précision importante. Durant une phase per-opératoire, après fixation des broches 61 et 62 dans l'os B à travers le bloc spécifique 10, l'organe 1144 est amovible du bloc spécifique 10 par déclipsage du plot 1135, de la patte 1148 et du téton 1184. Autrement dit, le dégagement mécanique réversible de la partie 30 peut être réalisé par simple déclipsage de l'organe 1144. Pour sa part, le déclipsage de l'organe 1143 n'est pas nécessaire, voire n'est pas souhaité en raison de la présence du plot 1125 muni de l'orifice 1127.

En service, les plots 1125 et 1135 sont utilisés pour le guidage d'un outil afin de percer l'os B. Autrement dit, les plots de guidage 1125 et 1135 sont portés directement par les moyens de dégagement mécanique 1140. De préférence, les organes de liaison 1143 et 1144 sont réalisés en métal. Lorsqu'un perçage est effectué dans les trous 1127 ou 1137 à travers les plots 1125 ou 1135, le risque de formation de débris ou copeaux est réduit, voire nul, quand les organes 1143 et 1144 sont en métal. Les plots 1125 et 1135 et les orifices 1127 et 1137 sont configurés avec une direction et une hauteur de coupe spécifique, en fonction du plan pré-opératoire spécifique à chaque patient.

En variante non représentée des moyens 1140, les plots 1125 et 1135 peuvent comporter des moyens de déplacement en translation et/ou en rotation pour le réglage de leur position et inclinaison par rapport au bloc spécifique 10.

Sur les figures 24 à 28 sont représentés des moyens de dégagement mécanique 1240 appartenant à un treizième mode de réalisation d'une instrumentation 1.

Un espace intermédiaire 1241 est ménagé entre les parties 20 et 30. Les moyens 1240 comprennent une boîte 1242 muni d'un compartiment de tiroir 1243 dans lequel une languette 1244 est susceptible de coulisser. La boite 1242 est solidaire de la partie 20. La languette 1244 est solidaire de la partie 30 et, initialement, reliée à une paroi 1245 de la boite 1242 par l'intermédiaire de pions allongés 1246. Des portions amincies 1247 des pions 1246 relient ces pions 1246 avec la languette 1244. Une paroi intermédiaire 1248 sépare le compartiment 1243 d'un compartiment 1249, lequel est agencé entre la paroi 1245 et la paroi 1248. Les pions 1246 s'étendent à travers des orifices 1276 ménagés à travers la paroi 1248.

Les moyens 1240 comprennent également un organe rotatif 1251 centré sur un axe A1 et s'étendant en partie hors de la boite 1242. Plus précisément, l'organe 1251 comporte une tête de manipulation 1252 située hors du la boite 1242 du côté de la surface 14, une hélice 1255 qui est située dans le compartiment 1249, ainsi qu'une portion 1253 qui relie la tête 1252 et l'hélice 1255 et s'étend suivant l'axe A1 à travers une ouverture 1275 ménagée dans la paroi 1245 de la boite 1242. L'hélice 1255 comprend des pales 1256 réparties radialement autour de l'axe A1. La portion 1253 comporte un évidement cylindrique 1254 centré sur l'axe A1, débouchant au niveau de l'hélice 1255 et non débouchant au niveau de la tête 1252. Un pion cylindrique 1261 s'étend depuis la languette 1244 suivant l'axe A1 en direction de la tête 1252, est rattaché à la languette 1244 par une portion amincie 1262 et est reçu dans l'évidement cylindrique 1254. Ainsi, le pion 1261 et la portion 1253 sont en liaison pivot autour de l'axe A1. Autrement dit, le pion 1261 est adapté pour supporter l'organe 1251 pivotant suivant un mouvement de rotation R1 autour de l'axe A1.

En pratique, le chirurgien fait pivoter l'organe 1251 autour de l'axe A1 suivant la rotation R1, en manipulant la tête 1252. L'organe 1251 pivote alors autour du pion 1261. Les pales 1256 de l'hélice 1255 viennent en contact avec les pions 1256 et exercent des efforts sur ces pions 1256 jusqu'à leur rupture, notamment au niveau des portions amincies 1247. Ainsi, la languette 1244 est désolidarisée des pions 1256 et donc de la boite 1242, et peut coulisser dans le compartiment 1243. A ce stade, le pion 1261 se désolidarise de la languette 1244 au niveau de la portion amincie 1262, soumise à un cisaillement entre la portion 1253 et la languette 1244. Autrement dit, le dégagement mécanique de la partie 30 solidaire de la languette 1244 est réalisé par rupture des pions 1256 lors de la rotation de l'hélice 1255, puis par coulissement de la languette 1244.

De préférence, les moyens de dégagement mécanique 1240 sont venus de matière à la fois avec la partie principale 20 et la partie secondaire 30. Dans le cas où les moyens 1240, qui présentent des formes complexes, sont fabriqués par frittage de poudre, alors la poudre résiduelle présente dans la boite 1242 doit être évacuée durant la phase pré-opératoire. A cet effet, la boite 1242 est pourvue d'orifices 1281 ménagés à travers sa paroi 1245, comme montré aux figures 24 et 28. En complément, la boite 1242 peut également comporter des orifices d'évacuation de poudre ménagés à travers des parois latérales 1291. Toutefois, la boite 1242 ne comporte pas d'orifices sur sa paroi opposée à la paroi 1245 et à la surface 14. En effet, lors de la rupture des pions 1256, les débris éventuels sont récupérés dans la boite 1242. Si la boite comportait des orifices ménagés à travers sa paroi opposée à la surface 14, les débris seraient susceptibles de s'échapper du côté de l'os B du patient, ce qu'il convient d'éviter.

Sur l'exemple non limitatif des figures 24 à 28, les pions 1246, les pales 1256 et les ouvertures 1276 sont au nombre de quatre. En alternative, les moyens 1240 peuvent être conformés différemment sans sortir du cadre de l'invention. Par exemple, les éléments 1246, 1256 et 1276 peuvent présenter un nombre différent. Selon un autre exemple, les moyens 1240 peuvent ne pas comporter de pion 1261.

Sur les figures 29 à 34 est représentée une instrumentation 1' conforme à un quatorzième mode de réalisation.

L'os B est schématisé sous forme d'une sphère et est recouvert de cartilage osseux C. En particulier, la direction d'observation de la figure 30 correspondant à la direction latéro-médiale d'un fémur, tandis que la direction antéro-postérieure est représentée par une flèche D1.

L'instrumentation 1' comprend un bloc spécifique 10', ainsi que des moyens de dégagement mécanique 40' ménagés sur le bloc spécifique 10'. Les autres éléments constitutifs de l'instrumentation 1' présentent un fonctionnement similaire, mais une structure différente, en comparaison avec les éléments constitutifs de l'instrumentation 1 du premier mode de réalisation des figures 1 à 5. Dans un but de simplification, ces éléments constitutifs 12, 14, 16, 20, 27, 28, 30, 37 et 38 comportent les mêmes références numériques que ceux du premier mode de réalisation. L'instrumentation 1' comprend également des broches d'ancrage osseux, seule la broche 63 étant représentée à la figure 32.

Le bloc 10' de l'instrumentation 1' comprend une partie principale 20 et une partie secondaire 30, toutes deux entièrement détachables de l'os B. La partie Le bloc 10' est spécialement conçu épouser le profil du cartilage C, qui présente des irrégularités, de comme visible aux figures 30, 32 et 34. Le bloc 10' ne peut donc pas être ajusté sur l'os B et retiré de cet os B de la même manière que le bloc 10 de l'instrumentation 1.

Dans ces conditions, les parties 20 et 30 sont disjointes. La zone de jonction 16 entre les parties 20 et 30 est délimitée au niveau des trous 27 et 28 de passage de broches. La fabrication des parties 20 et 30 est réalisée séparément, puis ces parties 20 et 30 sont assemblées par clipsage, grâce aux moyens 40'. En alternative, les deux parties 20 et 30 sont fabriquées ensemble puis détachées après coup. Afin de faciliter leur manipulation, les parties 20 et 30 comportent respectivement une poignée 29 et une poignée 39. La partie 20 vient se positionner sur l'os B et le cartilage C suivant la direction antéro-postérieure D1, tandis que la partie 30 est positionnée suivant la direction postéro-antérieure opposée à la direction D1.

Plus précisément, les moyens 40' comprennent une séparation 41' qui s'étend suivant un plan brisé dans la zone de jonction 16 et passe par les orifices 27 et 28. Les parties 20 et 30 sont adaptées pour venir en appui au niveau de la séparation 41'. Les moyens 40' comprennent également deux tiges 42' qui s'étendent depuis la partie 20 suivant la direction antéro-postérieure D1, ainsi que deux cavités 46' qui sont ménagées dans la partie 20 et sont prévues pour recevoir les tiges 42' suivant la direction D1. Les cavités 46' débouchent, d'une part, au niveau de la séparation 41' et, d'autre part, dans des ouvertures 47' ménagées au niveau de la surface 14, dans la partie 30. Des protubérances 43' sont formées sur les tiges 42', du côté des extrémités 44' des tiges 42' opposées à la partie 20, en saillie suivant une direction perpendiculaire à la direction D1. Les tiges 42' sont déformables élastiquement et tendent à s'écarter l'une de l'autre.

Lorsque les parties 20 et 30 sont assemblées pour former le bloc 10', les tiges 42' pénètrent dans les cavités 46', les extrémités 44' viennent se positionner dans les ouvertures 47', tandis que les protubérances 43' viennent se coincer contre les parois de ces ouvertures 47', formant ainsi une liaison par clipsage des tiges 42' dans la partie 30. Un appui des doigts du chirurgien sur les extrémités 44', en rapprochant ces deux extrémités 44', permet de déclipser les parties 20 et 30. Autrement dit, les moyens 40' équipant l'instrumentation 1' constituent à la fois des moyens d'engagement et de dégagement mécanique des parties 20 et 30 entre elles et avec l'os B. En particulier, les tiges 42' et les protubérances 43' constituent des éléments d'accroche temporaire de la partie 20 à la partie 30.

A la lecture de la description qui précède, l'homme du métier comprend que les moyens de dégagement mécanique 40 à 1240 et 40' des différents modes de réalisation peuvent être conformés différemment.

Quel que soit le mode de réalisation, les moyens de dégagement incluent au moins une cavité spécifique au patient. Par exemple, deux cavités peuvent être ménagés sur le bloc, selon une géométrie spécifique au patient. Les cavités peuvent être associées à des éléments favorisant la sécabilité et la rétention de débris. Lorsque le bloc est fabriqué par frittage de poudre, chaque cavité comporte au moins une ouverture débouchante hors du bloc, sur au moins une face de ce bloc. Chaque cavité est de préférence prévue selon une géométrie permettant le maintien d'une résistance mécanique satisfaisante suivant la direction d'appui du bloc vers l'os. Chaque cavité peut suivre une trajectoire correspond à un chemin de sécabilité ou de pliage particulier, le long duquel la résistance mécanique des moyens de dégagement est minimal, par exemple à proximité des plots de perçage.

En outre, les caractéristiques techniques des différents modes de réalisation peuvent être, en totalité ou pour certaines d'entre elles, combinées entre elles. Ainsi, l'instrumentation et les moyens de dégagement mécanique peuvent être adaptés en termes de simplicité de fabrication et d'utilisation, de coût et de performance. En particulier, les moyens de dégagement mécanique peuvent être réversibles ou irréversibles et adaptés aux préférences du chirurgien. De plus, les moyens de dégagement mécanique peuvent être adaptés pour la préparation d'un ossement donné du patient.

## Revendications

1. Instrumentation chirurgicale spécifique à un patient (1 ; 1') pour la préparation d'un os (B) du patient, l'instrumentation comprenant un bloc spécifique au patient (10 ; 10') délimitant une surface (12) d'appui fixe sur l'os (B), qui est conformée de manière spécifiquement ajustée à cet os (B), et incluant une partie principale (20) qui délimite en partie la surface d'appui fixe (12) et qui délimite au moins deux orifices primaires (27, 28) de passage respectif d'une broche primaire (61, 62), ces orifices primaires traversant le bloc (10 ; 10') entre la surface d'appui et une surface opposée (14) du bloc, l'instrumentation (1 ; 1') comprenant également :
- une partie secondaire (30) qui appartient au bloc (10 ; 10'), qui délimite en partie la surface d'appui (12) et qui comporte au moins deux orifices secondaires (37, 38) de passage respectif d'un outil de préparation osseuse (63, 64), tel qu'un foret de perçage ou une broche d'ancrage, ces orifices secondaires traversant le bloc entre la surface d'appui et la surface opposée (14) du bloc, et
- des moyens (40 ; 140 ; 240 ; 340 ; 440 ; 540 ; 640 ; 740 ; 840 ; 940 ; 1040 ; 1140 ; 1240 ; 40') de dégagement mécanique de la partie secondaire (30) par rapport à la partie principale (20), adaptés pour, alors que la partie principale est fixée sur l'os (B) par des broches primaires (61, 62) passées dans les orifices primaires (27, 28), rendre la partie secondaire (30) amovible par rapport à la partie principale,
l'instrumentation (1 ; 1') étant **caractérisée en ce que** les moyens de dégagement mécanique (40) comprennent au moins une cavité spécifique au patient (43, 44), qui s'étend à travers le bloc (10), entre la partie principale (20) et la partie secondaire (30), suivant une enveloppe géométrique de la surface d'appui fixe (12).

2. Instrumentation (1 ; 1') selon la revendication 1, **caractérisée en ce que** le bloc (10) et la cavité spécifique (43, 44) présentent chacun une épaisseur (e10 ; e43, e44) sensiblement constante, suivant une direction normale à la surface d'appui fixe (12), au niveau des moyens de dégagement mécanique (40).

3. Instrumentation (1) selon l'une quelconque des revendications 1 ou 2, **caractérisée en ce que** le bloc (10) comporte au moins une ouïe (45 ; 46) débouchant d'une part, dans la cavité spécifique (43 ; 44) et, d'autre part, au niveau de la surface d'appui fixe (12) ou de la surface opposée (14) du bloc (10).

4. Instrumentation (1) selon l'une quelconque des revendications précédentes, **caractérisée en ce que** les moyens de dégagement mécanique (40) comprennent deux cavités spécifiques au patient (43, 44), qui s'étendent à travers le bloc (10) suivant une enveloppe géométrique de la surface d'appui fixe (12).

5. Instrumentation (1) selon l'une quelconque des revendications précédentes, **caractérisée en ce que** la ou chaque cavité spécifique (43, 44) comporte au moins une ouverture débouchant hors du bloc (10).

6. Instrumentation (1) selon l'une quelconque des revendications précédentes, **caractérisée en ce que** les moyens de dégagement mécanique (40 ; 140 ; 240 ; 340 ; 440 ; 540 ; 640 ; 740 ; 840 ; 940 ; 1040 ; 1140 ; 1240 ; 40') sont en outre adaptés pour, alors que des broches secondaires (63, 64) sont passées dans les orifices secondaires (37, 38), rendre la partie secondaire (30) amovible par rapport à la partie principale (20) par coulissement le long des broches secondaires.

7. Instrumentation (1) selon l'une quelconque des revendications précédentes, **caractérisée en ce que** les moyens de dégagement mécanique (40 ; 140 ; 240 ; 340 ; 440 ; 540 ; 640 ; 740 ; 840 ; 1240) sont venus de matière à la fois avec la partie principale et la partie secondaire.

8. Instrumentation (1) selon l'une quelconque des revendications précédentes, **caractérisée en ce que** les moyens de dégagement mécanique (40 ; 140 ; 240 ; 340 ; 440 ; 540 ; 640 ; 740 ; 840 ; 1240) sont venus de matière à la fois avec la partie principale et la partie secondaire lors de la fabrication du bloc (10) par frittage laser de poudre.

9. Instrumentation (1) selon l'une quelconque des revendications précédentes, **caractérisée en ce que** les moyens de dégagement mécanique (40 ; 140 ; 340 ; 440 ; 540 ; 640 ; 740 ; 840 ; 1240) comprennent au moins un élément de liaison (41, 42 ; 142 ; 343, 343 ; 442 ; 544, 548 ; 646 ; 746 ; 842, 846 ; 1246, 1247) qui relie la partie principale (20) et la partie secondaire (30) et est destructible par rupture, par arrachement et/ou par section.

10. Instrumentation (1) selon la revendication 9, **caractérisée en ce que** les moyens de dégagement mécanique (40 ; 640 ; 740 ; 840) comprennent au moins une ouverture (45, 46 ; 643, 645 ; 745 ; 845) de réception d'un outil au contact du ou des éléments de liaison (41, 42 ; 646 ; 746 ; 842, 846) pour la rupture, l'arrachement et/ou la section du ou des éléments de liaison.

11. Instrumentation (1) selon l'une quelconque des revendications 1 à 8, **caractérisée en ce que** les moyens de dégagement mécanique (240 ; 540) comprennent un élément déformable élastiquement (242 ; 542) qui relie la partie principale (20) et la partie secondaire (30) et autorise un dégagement réversible par pliage de la partie secondaire (30) par rapport à la partie principale (20).

12. Instrumentation (1) selon la revendication 9, **caractérisée en ce que** les moyens de dégagement mécanique (1240) comprennent un organe rotatif (1251) et **en ce qu'**une rotation (R1) de l'organe rotatif (1251) exerce des efforts sur le ou les éléments de liaison (1246, 1247) pour la rupture, l'arrachement et/ou la section du ou des éléments de liaison.

13. Instrumentation (1) selon l'une quelconque des revendications 1 à 6, **caractérisée en ce que** les moyens de dégagement mécanique (940 ; 1040) sont adaptés pour successivement être solidarisés au bloc (10) et au moins partiellement désolidarisés du bloc (10), notamment par clipsage/déclipsage ou par vissage/dévissage.

14. Instrumentation (1') selon l'une quelconque des revendications 1 à 6, **caractérisée en ce que** les moyens de dégagement mécanique (40') sont au moins partiellement venus de matière avec une première partie parmi la partie principale (20) et la partie secondaire (30).

15. Instrumentation (1') selon la revendication 14, **caractérisée en ce que** les moyens de dégagement mécanique (40') comportent au moins un élément (42', 43') d'accroche temporaire de la première partie sur une deuxième partie parmi la partie principale (20) et la partie secondaire (30).

## Patentansprüche

1. Für einen Patienten spezifische chirurgische Instrumentierung (1; 1') für die Vorbereitung eines Knochens (B) des Patienten, wobei die Instrumentierung einen für den Patienten spezifischen Block (10; 10') aufweist, der eine feste Auflagefläche (12) an dem Knochen (B) begrenzt, die auf spezifisch passende Weise an den Knochen (B) angepasst ist, und einen Hauptabschnitt (20) aufweist, der die feste Auflagefläche (12) teilweise begrenzt und mindestens zwei Hauptöffnungen (27, 28) für ein jeweiliges Durchlassen eines Hauptstiftes (61, 62) begrenzt, wobei die Hauptöffnungen den Block (10, 10') zwischen der Auflagefläche und einer entgegengesetzten Fläche (14) des Blocks durchqueren, wobei die Instrumentierung (1, 1') ferner aufweist:
- einen Nebenabschnitt (30), der zu dem Block (10, 10') gehört, der die Auflagefläche (12) teilweise begrenzt und mindestens zwei Nebenöffnungen (37, 38) für ein jeweiliges Durchlassen eines Werkzeug zur Knochenvorbereitung (63, 64), wie zum Bespiel einen Bohreinsatz oder einen Verankerungsstift aufweist, wobei die Nebenöffnungen den Block zwischen der Auflagefläche und der entgegengesetzten Fläche (14) des Blocks durchqueren, und
- Mittel (40, 140, 240, 340, 440, 540, 640, 740, 840, 940, 1040, 1140, 1240, 40') zum mechanischen Lösen des Nebenabschnitts (30) bezüglich des Hauptabschnitts (20), die angepasst sind, den Nebenabschnitt (30) bezüglich des Hauptabschnitts lösbar zu machen, wenn der Hauptabschnitt mittels in die Hauptöffnungen (27, 28) eingesetzter Hauptstifte (61, 62) an dem Knochen (B) befestigt ist,
wobei die Instrumentierung (1, 1') **dadurch gekennzeichnet ist, dass** die Mittel zum mechanischen Lösen (40) mindestens einen für den Patienten spezifischen Hohlraum (43, 44) aufweisen, der sich durch den Block (10) hindurch zwischen dem Hauptabschnitt (20) und dem Nebenabschnitt (30) entlang einer geometrischen Ummantelung der ortsfesten Auflagefläche (12) erstreckt.

2. Instrumentierung (1, 1') gemäß Anspruch 1, **dadurch gekennzeichnet, dass** der Block (10) und der spezifische Hohlraum (43, 44) jeweils eine im Wesentlichen konstante Dicke (e10, e43, e44) entlang einer zu der festen Auflagefläche (12) senkrechten Richtung auf der Höhe der Mittel zum mechanischen Lösen (40) haben.

3. Instrumentierung (1) gemäß irgendeinem der Ansprüche 1 oder 2, **dadurch gekennzeichnet, dass** der Block (10) mindestens einen Luftschlitz (45, 46) aufweist, der einerseits in den spezifischen Hohlraum (43, 44) und andererseits auf der Höhe der festen Auflagefläche (12) oder der entgegengesetzten Fläche (14) des Blocks (10) einmündet.

4. Instrumentierung (1) gemäß irgendeinem der vorhergehenden Ansprüche, **dadurch gekennzeichnet, dass** die Mittel zum mechanischen Lösen (40) zwei für den Patienten spezifische Hohlräume (43, 44) aufweisen, die sich durch den Block (10) hindurch entlang einer geometrischen Ummantelung der festen Auflagefläche (12) erstrecken.

5. Instrumentierung (1) gemäß irgendeinem der vorhergehenden Ansprüche, **dadurch gekennzeichnet, dass** der oder jeder spezifische Hohlraum (43, 44) mindestens eine Öffnung aufweist, die außerhalb des Blocks (10) mündet.

6. Instrumentierung (1) gemäß irgendeinem der vorhergehenden Ansprüche, **dadurch gekennzeichnet, dass** die Mittel zum mechanischen Lösen (40, 140, 240, 340, 440, 540, 640, 740, 840, 940, 1040, 1140, 1240, 40') ferner angepasst sind, den Nebenabschnitt (30) bezüglich des Hauptabschnitts (20) durch Gleiten entlang den Nebenstiften lösbar zu machen, wenn die Nebenstifte (63, 64) in die Nebenöffnungen (37, 38) eingesetzt sind.

7. Instrumentierung (1) gemäß irgendeinem der vorhergehenden Ansprüche, **dadurch gekennzeichnet, dass** die Mittel zum mechanischen Lösen (40, 140, 240, 340, 440, 540, 640, 740, 840, 1240) sowohl mit dem Hauptabschnitt als auch mit dem Nebenabschnitt aus einem Stück sind.

8. Instrumentierung (1) gemäß irgendeinem der vorhergehenden Ansprüche, **dadurch gekennzeichnet, dass** die Mittel zum mechanischen Lösen (40, 140, 240, 340, 440, 540, 640, 740, 840, 1240) bei der Herstellung des Blocks (10) mittels Laserpulversinterns sowohl mit dem Hauptabschnitt als auch mit dem Nebenabschnitt aus einem Stück sind.

9. Instrumentierung (1) gemäß irgendeinem der vorhergehenden Ansprüche, **dadurch gekennzeichnet, dass** die Mittel zum mechanischen Lösen (40, 140, 340, 440, 540, 640, 740, 840, 1240) mindestens ein Verbindungselement (41, 42, 142, 343, 343, 442, 544, 548, 646, 746, 842, 846, 1246, 1247) aufweisen, das den Hauptabschnitt (20) und den Nebenabschnitt (30) miteinander verbindet und durch Brechen, Beißen und/oder Sektion zerstörbar ist.

10. Instrumentierung (1) gemäß Anspruch 9, **dadurch gekennzeichnet, dass** die Mittel zum mechanischen Lösen (40, 640, 740, 840) mindestens eine Öffnung (45, 46, 643, 645, 745, 845) zum Aufnehmen eines Werkzeugs in Kontakt mit dem oder den Verbindungsmitteln (41, 42, 646, 746, 842, 846) für das Brechen, Reißen und/oder die Sektion des oder der Verbindungselemente aufweisen.

11. Instrumentierung (1) gemäß irgendeinem der Ansprüche 1 bis 8, **dadurch gekennzeichnet, dass** die Mittel zum mechanischen Lösen (240, 540) ein elastisch verformbare Element (242, 542) aufweisen, das den Hauptabschnitt (20) und den Nebenabschnitt (30) miteinander verbindet und ein reversibles Lösen durch Falten des Nebenabschnitts (30) bezüglich des Hauptabschnitts (20) zulässt.

12. Instrumentierung (1) gemäß Anspruch 9, **dadurch gekennzeichnet, dass** die Mittel zum mechanischen Lösen (1240) ein Rotationsmittel (1251) aufweisen, und dass eine Rotation (R1) des Rotationsmittels (1251) Kräfte auf das oder die Verbindungselemente (1246, 1247) für das Brechen, Reißen und/oder die Sektion des oder der Verbindungselemente ausübt.

13. Instrumentierung (1) gemäß irgendeinem der Ansprüche 1 bis 6, **dadurch gekennzeichnet, dass** die Mittel zum mechanischen Lösen (940, 1040) angepasst sind, nacheinander am Block (10) gesichert zu sein und zumindest teilweise von dem Block (10) gelöst zu sein, insbesondere durch Klemmverbinden/Lösen der Klemmverbindung oder durch Schrauben/Lösen der Schraubverbindung.

14. Instrumentierung (1') gemäß irgendeinem der Ansprüche 1 bis 6, **dadurch gekennzeichnet, dass** die Mittel zum mechanischen Lösen (40') zumindest teilweise aus einem Stück mit einem ersten Abschnitt von dem Hauptabschnitt (20) und dem Nebenabschnitt (30) sind.

15. Instrumentierung (1') gemäß Anspruch 14, **dadurch gekennzeichnet, dass** die Mittel zum mechanischen Lösen (40') mindestens ein Element (42', 43') zum vorübergehenden Einhaken des ersten Abschnitts an einem zweiten Abschnitt von dem Hauptabschnitt (20) und dem Nebenabschnitt (30) aufweisen.

## Claims

1. Surgical instrumentation specific to a patient (1; 1') for preparing a bone (B) of the patient, the instrumentation comprising a patient-specific block (10; 10') delimiting a fixed supporting surface (12) on the bone (B), which is shaped so as to be specifically fitted to this bone (B), and including a main portion (20) which partly delimits the fixed supporting surface (12) and which delimits at least two primary orifices (27, 28) for respectively letting through a primary pin (61, 62), these primary orifices passing through the block (10; 10') between the supporting surface and an opposite surface (14) of the block,
the instrumentation (1; 1') also comprising:
- a secondary portion (30) which belongs to the block (10 ; 10'), which partly delimits the supporting surface (12) and which includes at least two secondary orifices (37, 38) for respectively letting through a bone preparation tool (63, 64), such as a boring bit or an anchoring pin, these secondary orifices crossing the block between the supporting surface and the opposite surface (14) of the block, and
- means (40; 140; 240; 340; 440; 540; 640; 740; 840; 940; 1040; 1140; 1240; 40') for mechanically disengaging the secondary portion (30) with respect to the main portion (20), suitable for, while the main portion is attached onto the bone (B) by primary pins (61, 62) having passed into the primary orifices (27, 28), making the secondary portion (30) removable with respect to the main portion,
the instrumentation (1; 1 being **characterized in that** the mechanical disengagement means (40) comprise at least one patient-specific cavity (43, 44), which extends through the block (10) between the main portion (20) and the secondary portion (30), along a geometrical envelope of the fixed supporting surface (12).

2. The instrumentation (1; 1') according to claim 1, **characterized in that** the block (10) and the specific cavity (43, 44) each have a substantially constant thickness (e10; e43, e44), along a direction normal to the fixed supporting surface (12), at the mechanical disengagement means (40).

3. The instrumentation (1) according to any of claims 1 or 2, **characterized in that** the block (10) includes at least one vent (45; 46) opening into the specific cavity (43; 44) on the one hand and at the fixed supporting surface (12) or the opposite surface (14) of the block (10) on the other hand.

4. The instrumentation (1) according to any of the preceding claims, **characterized in that** the mechanical disengagement means (40) comprise two cavities (43, 44) specific to this patient, which extend through the block (10) along a geometrical envelope of the fixed supporting surface (12).

5. The instrumentation (1) according to any of the preceding claims, **characterized in that** said or each specific cavity (43, 44) includes at least one aperture opening outside the block (10).

6. The instrumentation (1) according to any of the preceding claims, **characterized in that** the mechanical disengagement means (40; 140; 240; 340; 440; 540; 640; 740; 840; 940; 1040; 1140; 1240; 40') are further suitable for, while the secondary pins (63, 64) are passed into the secondary orifices (37, 38), making the secondary portion (30) removable with respect to the main portion (20) by sliding along the secondary pins.

7. The instrumentation (1) according to any of the preceding claims, **characterized in that** the mechanical disengagement means (40; 140; 240; 340; 440; 540; 640; 740; 840; 940; 1040; 1140; 1240;) are made both with the main portion and the secondary portion in the same material.

8. The instrumentation (1) according to any of the preceding claims, **characterized in that** the mechanical disengagement means (40; 140; 240; 340; 440; 540; 640; 740; 840; 940; 1040; 1140; 1240;) are made both with the main portion and the secondary portion in the same material during the manufacturing of the block (10) by laser powder sintering.

9. The instrumentation (1) according to any of the preceding claims, **characterized in that** the mechanical disengagement means (40; 140; 240; 340; 440; 540; 640; 740; 840; 940; 1040; 1140; 1240;) comprise at least one connecting element (41, 42 ; 142 ; 343, 343 ; 442 ; 544, 548 ; 646 ; 746 ; 842, 846; 1246, 1247) which connects the main portion (20) and the secondary portion (30) and which may be destroyed by breakage, tearing off and/or by severance.

10. The instrumentation (1) according to claim 9, **characterized in that** the mechanical disengagement means (40; 640; 740; 840) comprise at least one aperture (45, 46; 643, 645; 745; 845) for receiving a tool in contact with the connecting element(s) (41, 42; 646; 746; 842, 846) for breaking, tearing off and/or severing the connecting element(s).

11. The instrumentation (1) according to any of claims 1 to 8, **characterized in that** the mechanical disengagement means (240; 540) comprise an elastically deformable element (242; 542) which connects the main portion (20) and the secondary portion (30) and allows reversible disengagement by folding the secondary portion (30) with respect to the main portion (20).

12. The instrumentation (1) according to claim 9, **characterized in that** the mechanical disengagement means (1240) comprise a rotary member (1251) and **in that** a rotation (R1) of the rotary member (1251) exerts forces on the connecting element(s) (1246, 1257) for tearing off and/or severing the connecting element(s).

13. The instrumentation (1) according to any of claims 1 to 6, **characterized in that** the mechanical disengagement means (940; 1040) are adapted so as to be successively secured to the block (10) and then at least partly detached from the block (10), notably by clipping/unclipping or by screwing/unscrewing.

14. The instrumentation (1') according to any of claims 1 to 6, **characterized in that** the mechanical disengagement means (40') are at least partly made with a first portion from among the main portion (20) and a secondary portion (30), in the same material.

15. The instrumentation (1') according to claim 14, **characterized in that** the mechanical disengagement means (40') include at least one element (42', 43') for having the first portion temporarily adhere onto a second portion from among the main portion (20) and the secondary portion (30).
